(19) Europäisches Patentamt
European Patent Office
Office européen des brevets

(11) Veröffentlichungsnummer: **0 184 982**
**B1**

(12) **EUROPÄISCHE PATENTSCHRIFT**

(45) Veröffentlichungstag der Patentschrift: **23.01.91**

(21) Anmeldenummer: **85810505.9**

(22) Anmeldetag: **01.11.85**

(51) Int. Cl.$^5$: **C 07 D 487/04,**
C 07 D 207/38, C 08 K 5/00

# (54) Verfahren zur Herstellung von Pyrrolo-3,4-cr-pyrrolen und neue Pyrrolo-3,4-cr-pyrrole.

(30) Priorität: **07.11.84 CH 5336/84**

(43) Veröffentlichungstag der Anmeldung:
**18.06.86 Patentblatt 86/25**

(45) Bekanntmachung des Hinweises auf die Patenterteilung:
**23.01.91 Patentblatt 91/04**

(84) Benannte Vertragsstaaten:
**CH DE FR GB IT LI**

(56) Entgegenhaltungen:
**EP-A-0 061 426**
**EP-A-0 094 911**
**EP-A-0 098 808**
**EP-A-0 133 156**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Pfenninger, Johannes, Dr.**
**Route du Confin 23**
**CH-1723 Marly (CH)**
Erfinder: **Iqbal, Abul, Dr.**
**Im Schaiengarten 1**
**CH-4107 Ettingen (CH)**
Erfinder: **Rochat, Alain Claude, Dr.**
**Route de Schiffenen 38**
**CH-1700 Fribourg (CH)**
Erfinder: **Wallquist, Olof, Dr.**
**Route des Pralettes 23**
**CH-1723 Marly (CH)**

(56) Entgegenhaltungen:
**TETRAHEDRON LETTERS, Nr. 29, Juli 1974, Seiten 2549-2552, Pergamon Press, Oxford, GB; D.G. FARNUM et al.: "Attempted reformatskii reaction of benzonitrile, 1,4-diketo-3,6-diphenylpyrrolo3,4-Crpyrrole. A lactam analogue of pentalene"**

**TETRAHEDRON LETTERS, vol. 23, no. 15, 1982, Seiten 1598-1600, Pergamon Press, Oxford, GB; T. HIYAMA et al.: "A new synthesis of 3-amino-2-alkenoates"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 184 982 B1

## Beschreibung

Als Pigmente geeignete Pyrrolo-[3,4-c]-pyrrole und Verfahren zu ihrer Herstellung sind z.B. aus EP—A—0 061 426, EP—A—0 094 911 und EP—A—0 098 808 bekannt. Gemäss der in beiden ersten obengenannten Publikationen beschriebenen Verfahren ist es nicht möglich, reine asymmetrische Pyrrolo-[3,4-c]-pyrrole zu erhalten. Es entsteht immer ein erheblicher Anteil an symmetrischem Produkt. Ein erstes Verfahren, welches es erlaubt, gezielt und einheitlich asymmetrische 1,4-Diketopyrrolo-[3,4-c]-pyrrole der Formel

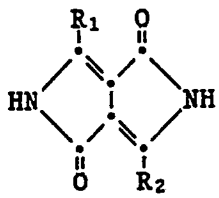

(1),

herzustellen, worin $R_1$ und $R_2$ aromatische Reste bedeuten, ist in EP—A—0 098 808 beschrieben. Die Ausbeute dieses Verfahrens ist allerdings unbefriedigend und ausserdem können dadurch praktisch keine Pyrrolo-[3,4-c]-pyrrole der obenerwähnten Formel hergestellt werden, worin mindestens einer der Reste $R_1$ und $R_2$ Alkykl, Aralkyl oder ortho-substituiertes Phenyl bedeutet.

Es ist nun neues Verfahren gefunden worden, das es überasschenderweise erlaubt, asymmetrische Pyrrolo-[3,4-c]-pyrrole mit einer unerwartet besseren Ausbeute zu erhalten und wodurch sogar auch solche Pyrrolo-[3,4-c]-pyrrole der obenerwähnten Formel hergestellt werden können, worin $R_1$ und/oder $R_2$ Alkyl, Aralkyl oder ortho-substituiertes Phenyl bedeuten.

Die Erfindung betrifft demnach ein Verfahren zur Herstellung von Pyrrolo-[3,4-c]-pyrrolen der Formel (1)

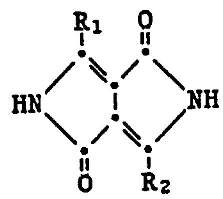

(1),

worin $R_1$ und $R_2$ $C_1$—$C_{18}$-Alkyl, Cyclohexyl, Benzyl, Phenyläthyl, Diphenylyl, Naphthyl, einen heterocyclischen Rest aus der Gruppe bestehend aus Pyridyl, Pyrimidyl, Pyrazinyl, Triazinyl, Furanyl, Pyrrolyl, Thiophenyl, Chinolyl, Cumarinyl, Benzfuranyl, Benzimidazolyl und Benzoxazolyl oder eine Gruppe

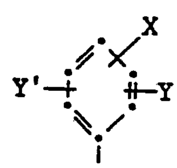

bedeuten, worin X, Y und Y' H- oder Halogenatome, Carbamoyl-, Trifluormethyl-, Cyan- oder $C_2$—$C_6$-Alkylcarbamoylgruppen, Alkyl-, Alkoxy- oder Alkylmercaptogruppen mit 1—6 C, Alkoxycarbonyl- oder Alkanoylamino- oder Dialkylaminogruppen mit 2—6 C, gegebenenfalls durch Halogen, Alkyl oder Alkoxy mit 1—6 C substituierte Phenoxy-, Phenylmercapto, Phenoxycarbonyl-, Phenylcarbamoyl- oder Benzoylaminogruppen bedeuten dadurch gekennzeichnet, dass man einen Ester der Formeln (2) oder (3)

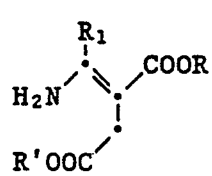

(2)

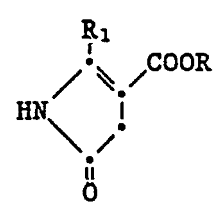

(3)

mit einem Nitril der Formel $R_2CN$ (4) in Gegenwart von 0,1 bis 10 Mol einer starken Base, bezogen auf 1 Mol des Reaktenden der Formel (2) oder (3), in einem organischen Lösungsmittel bei einer Temperatur von 60 bis 140°C umsetzt, wobei R und R' Alkylgruppen von 1—18 C-Atomen, unsubstituiertes oder durch

Methoxy oder Aethoxy substituiertes Phenyl bedeuten und $R_1$ und $R_2$ die bereits angegebene Bedeutung haben.

Bedeuten R, R', $R_1$ oder $R_2$ in den Formeln (1), (2) und (3) oder $R_2$ in der Formel (4) $C_1$—$C_{18}$-Alkylgruppen, dann enthalten sie insbesondere 1—12 und besonders bevorzugt 1—6 C-Atome. Als Beispiele seien Methyl, Aethyl, Isopropyl, sek.-Butyl, tert.-Butyl, tert.-Amyl und Octyl, Decyl, Dodecyl oder Stearyl genannt.

Zu den Pyrrolinonen der Formel (3) gelange man zum Beispiel durch Cyclisierung der Verbindung der Formel (2) in Gegenwart einer starken Base. Die Cyclisierung wird nach an sich bekannten Verfahren, z.B. mit Natriummethylat in Methanol bei Rückflusstemperatur durchgeführt.

Die Aminoester der Formel (2) ihrerseits erhält man zum Beispiel durch Umsetzen eines Bernsteinsäurediesters $R'OOCH_2CH_2COOR$ (5) mit einem Nitril $R_1CN$, wobei R, R' und $R_1$ die angegebene Bedeutung haben, in Gegenwart einer starken Base und Zink- oder Magnesiumsalzen analog den in Chem. Lett. *1982,* S. 687 und Tetr. Lett. *1982,* S. 1597 beschriebenen Verfahren.

Die Pyrrolinone der Formel (3) erhält man auch nach bekannten Verfahren durch Cyclisierung einer Verbindung der Formel (6)

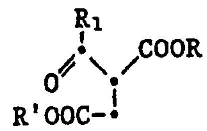

(6)

z.B. mit Ammoniumsalzen.

Die Verbindungen der Formel (6) sind bekannt und können z.B. durch Kondensation eines Acylessigesters der Formel (7)

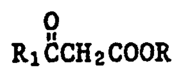

(7),

worin $R_1$ und R die angegebene Bedeutung haben, mit einem Ester der Formel $XCH_2COOR'$, wobei X ein Fluor-, Chlor-, Brom- oder Jodatom bedeutet und R' die angegebene Bedeutung hat, erhalten werden (Vgl. W. H. Perkin J. Chem. Soc. *47,* S. 262 oder Org. Synth. *42,* 75 (1962)).

Als Beispiele der im erfindungsgemässen Verfahren als Ausgangsstoffe zu verwendenden Nitrile seien genannt: Acetonitril, Propionitril, Butyronitril, Isobutyronitril, Hexylcyanid, Cyclohexylcyanid, Benzylcyanid, Benzonitril, o-, m- oder p-Chlorbenzonitril, o-, m- oder p-Brombenzonitril, o-, m- oder p-Methylbenzonitril, p-tert.-Butylbenzonitril, p-Phenylbenzonitril, o-, m- oder p-Methoxybenzonitril, p-Phenoxybenzonitril, 3,4-Dimethylbenzonitril, Isophthalonitril, Terephthalonitril, 3- oder 4-Pyrimidylcyanid.

Im erfindungsgemässen Verfahren zur Herstellung der Pyrrolopyrrole der Formel (1) verwendet man beispielsweise die in EP—A—94911 erwähnten Lösungsmittel. Bevorzugt werden Alkohole, insbesondere sekundäre oder tertiäre Alkohole. Bevorzugte tert.-Alkohole sind tert.-Butanol und tert.-Amylalkohol.

Das erfindungsgemässe Verfahren wird in Gegenwart einer starken Base durchgeführt. Geeignete starke Basen sind z.B. Alkalihydroxide, wie Natrium-, Kalium- oder Lithiumhydroxid, oder Erdalkalihydroxide, wie Calcium- oder Magnesiumhydroxid, oder Alkaliamide, wie Lithium- oder Lithiumdiisopropyl-, Lithiumdiäthyl- oder Lithiumisopropyl-cyclohexylamid, Natriumamid, oder Alkalihydride, wie Lithiumhydrid oder Natriumhydrid, oder Erdalkali oder Alkalialkoholate, die sich insbesondere von primären, sekundären oder tertiären aliphatischen Alkoholen mit 1 bis 10 C-Atomen ableiten, wie z.B. Natrium-, Kalium- oder Lithiummethylat, -äthylat, -n-propylat, -isopropylat, -n-butylat, -sek.-butylat, -tert.-butylat, -2-methyl-2-butylat, -2-methyl-2-pentylat, -3-methyl-3-pentylat, -3-äthyl-3-pentylat, oder Erdalkali- oder Alkaliphenolate oder o-alkylsubstituierte Phenolate wie Natrium- oder Kalium-o-kresolat. Man kann aber auch ein Gemisch der genannten Basen verwenden.

Im erfindungsgemässen Verfahren verwendet man als starke Base bevorzugt Alkalialkoholate, wobei Alkali insbesondere Natrium oder Kalium bedeutet, und das Alkoholat sich bevorzugt von einem sekundären oder tertiären Alkohol ableitet. Besonders bevorzugte starke Basen sind daher z.B. Natrium- oder Kalium-isopropylat, -sek.-butylat, -tert.-butylat und -tert.-amylat. Dabei können die Alkalialkoholate auch in situ hergestellt werden, indem man den entsprechenden Alkohol mit dem Alkalimetall, Alkalihydrid oder Alkaliamid umsetzt.

Im erfindungsgemässen Verfahren wird die starke Base bevorzugt in einer Menge von 0,9 bis 4,0 Mol, bezogen auf 1 Mol des Reaktanden der Formel (2) oder (3) eingesetzt.

Die genannten starken Basen können zusammen mit einem Phasentransferkatalysator eingesetzt werden. Dies ist vorallem dann von Vorteil, wenn die Löslichkeit einer bestimmten Base in einem bestimmten Lösungsmittel gering ist. Die Phasentransferkatalysatoren können in einer Menge von 0,001 bis 50 Mol-%, vorzugsweise 0,01 bis 0,3 Mol-%, bezogen auf die Reaktanden eingesetzt werden. Für das erfindungsgemässe Verfahren eignen sich die üblichen, in der Literatur beschriebenen Phasentransferkatalysatoren, wie sie z.B. in CHEMTECH, Februar 1980, S. 111, Tabelle 1, aufgeführt sind, nämlich beispielsweise quaternäre Salze, cyclische Polyäther, offenkettige Polyäther, N-Alkylphosphoramide mit

Methylen, überbrückte Phosphor- oder Schwefeloxide oder Salze von Sulfobernsteinsäureestern.

Die Umsetzungen werden vorzugsweise bei einer Temperatur von 80 bis 120°C, durchgeführt.

Es ist möglich, die Umsetzungen kontinuierlich durchzuführen.

Verwendet man als Lösungsmittel einen Alkohol und als Base ein Alkoholat, so kann es vorteilhaft sein, einen Alkohol und ein Alkoholat mit gleichen Alkylresten zu wählen. Ebenso vorteilhaft kann es sein, wenn zudem noch der Ester der Formel (2) oder (3) ebensolche Alkylgruppen enthält.

Je nach verwendeter Base fallen die Pyrrolopyrrole in Form ihrer Natrium- oder Kaliumsalze an, aus welchen durch Hydrolyse die Pyrrolopyrrole gewonnen werden.

Zur Hydrolyse des Kondensationsproduktes kann man eine Säure, einen Alkohol mit 1 bis 4 C-Atomen, wie Methanol oder Aethanol, vorzugsweise aber Wasser, verwenden. Als Säuren kommen z.B. aliphatische oder aromatische Carbon- oder Sulfonsäuren in Betracht, wie beispielsweise Ameisensäure, Essigsäure, Propionsäure, Oxalsäure, Benzoesäure oder Benzolsulfonsäure. Weiterhin kommen auch Mineralsäuren in Betracht, wie Chlorwasserstoff, dessen wässrige Lösung, sowie Kohlensäure, verdünnte Schwefel- oder Phosphorsäure.

Bei der Hydrolyse fällt die Verbindung der Formel (1) aus und kann durch Abfiltrieren isoliert werden.

Die nach dem erfindungsgemässen Verfahren erhältlichen Pyrrolo-(3,4-c]-pyrrole stellen wertvolle Pigmente dar, die zum Färben von hochmolekularem organischem Material verwendet werden können.

Je nach Verwendungszweck kann man die nach dem erfindungsgemässen Verfahren anfallenden Pigmente in eine deckendere oder transparentere Form überführen.

Um eine transparente Form zu erzielen, wird die Hydrolyse bevorzugt bei tieferen Temperaturen (unter 80°C) durchgeführt.

Wird eine deckendere Pigmentform gewünscht, so erweist sich eine Hydrolyse bei höherer Temperatur (über 80°C) gegebenenfalls unter Druck, als zweckmässig. Man kann auch das Pigment nach der Hydrolyse zuerst isolieren und nachträglich in Wasser oder in einem organischen Lösungsmittel erwärmen, gegebenenfalls unter Druck, um die deckende Form zu erlangen. Vorzugsweise verwendet man solche organische Lösungsmittel, die über 80°C sieden. Als besonders geeignet erweisen sich durch Halogenatome, Alkyl- oder Nitrogruppen substituierte Benzole, wie Xylole, Chlorbenzol, o-Dichlorbenzol oder Nitrobenzol, sowie Pyridinbasen, wie Pyridin, Picolin oder Chinolin, ferner Ketone, wie Cyclohexanon, Aether, wie Aethylenglykolmonomethyl- oder -monoäthyläther, Amide, wie Dimethylformamid oder N-Methylpyrrolidon, sowie Dimethylsulfoxid oder Sulfolan. Man kann die Nachbehandlung auch in Wasser, gegebenenfalls unter Druck, in Gegenwart von organischen Lösungsmitteln und/oder mit Zusatz von oberflächenaktiven Substanzen durchführen.

Hochmolekulare organische Materialien, die mit den nach dem erfindungsgemässen Verfahren erhältlichen Pyrrolo-[3,4-c]-pyrrolen gefärbt bzw. pigmentiert werden können, sind z.B. Celluloseäther und -ester, wie Aethylcellulose, Nitrocellulose, Celluloseacetat, Cellulosebutylat, natürliche Harze oder Kunstharze, wie Polymerisationsharze oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die nach dem erfindungsgemässen Verfahren erhältlichen als Pigmente einzusetzende Pyrrolo-[3,4-c]-pyrrole als Toner oder in Form von Präparaten einzusetzen. Bezogen auf das zu pigmentierende hochmolekulaire organische Material setzt man die nach dem erfindungsgemässen Verfahren erhältlichen Verbindungen der Formel (1) in einer Menge von vorzugsweise 0,1 bis 10 Gew.% ein.

Die erhaltenen Färbungen, beispielsweise in Kunststoffen, Fasern, Lacken oder Drucken, zeichnen sich durch hohe Farbstärke, gute Dispergierbarkeit, gute Ueberlackier-, Migrations-, Hitze-, Licht- und Wetterechtheit, sowie durch einen guten Glanz aus.

Die folgenden Beispiele erläutern die Erfindung. Temperaturen sind in °C angegeben.

### Beispiel 1

1,5 g Natrium und 30 mg des Na-Salzes des Sulfobernsteinsäure-bis-2-äthylhexylester werden bei Rückflusstemperatur in 30 ml tert.-Amylalkohol bis zur vollständigen Umsetzung gerührt. Zur klaren Lösung lässt man 2,2 des Pyrrolinons der Formel (9)

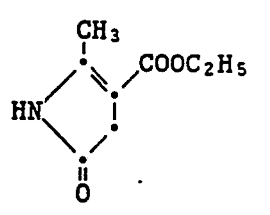

(9)

[hergestellt nach Ann. *260,* S. 137 (1890)], gelöst in 20 ml Acetonitril zutropfen, wobei die Reaktionstemperatur von 100° auf 80° fällt. Nach 1 Stunde Erhitzen bei Rückflusstemperatur lässt man

15 ml Wasser zutropfen und die Reaktionsmischung wird auf 40° abgekühlt. Das zweiphasige Gemisch wird in Aethylacetat aufgenommen und mit 1 N HCl sauer gestellt, worauf in der wässrigen Phase gelbe Kristalle ausfallen. Man trennt die wässrige Phase ab, filtriert und wäscht die erhaltenen Kirstalle mit Methanol und Wasser. Nach dem Trocknen im Vakuum werden 300 mg (14%) der Verbindung der Formel (10)

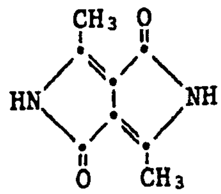

(10)

in Form gelber Kristalle isoliert.

Smp.: >250°; UV ($CH_3OH$, $\lambda_{max}$, ε) 380 (14500), 392 (15800).

| | | | | |
|---|---|---|---|---|
| $C_8H_8N_2O_2$ | Ber.: | C 58,53 | H 4,91 | N 17,06 |
| | Gef.: | C 57,80 | H 5,11 | N 16,81. |

Beispiel 2

1 g Natrium und 0.02 g Sulfobernsteinsäure-bis-2-äthylhexylester Natriumsalz (Emulgator) werden bei Rückflusstemperatur in 20 ml tert.-Amylalkohol bis zur vollständigen Umsetzung gerührt. Zur klaren Lösung gibt man 3 ml n-Butyronitril und anschliessend während 10 Min. portionenweise 1 g der Verbindung der Formel (9). Das Reaktionsgemisch wird 1 Stunde am Rückfluss gekocht. Man kühlt auf 70° ab und lässt über 20 Min. ein Gemisch von 5 ml Eissessig und 7 ml Methanol zutropfen. Man nimmt in Essigester auf und wäscht mit konz. Kochsalz-Lösung. Die mit Natriumsulfat getrocknete erhaltene Lösung wird eingeengt, wobei sich eine gelbe Fällung bildet. Man filtriert ab, wäscht mit wenig Methylenchlorid und trocknet im Vakuum. Das erhaltene Pigment der Formel (11) (270 mg, 24%) ergibt, in PVC eingearbeitet, eine gelbe Färbung

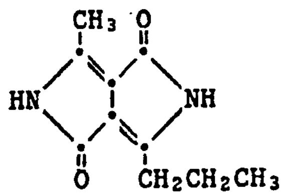

(11)

Smp.: >250°.

UV, VIS ($CH_3OH$, $\lambda_{max}$, ε) 382 (12800), 398 (14000).

| | | | | |
|---|---|---|---|---|
| $C_{10}H_{12}N_2O_2$ | Ber.: | C 62,49 | H 6,29 | N 14,57 |
| | Gef.: | C 61,21 | H 6,66 | N 13,41. |

Beispiel 3

2 g Natrium und 100 mg des Na-Salzes des Sulfobernsteinsäure-bis-2-äthylhexylesters werden bei Rückflusstemperatur bis zur vollständigen Umsetzung gerührt. Man lässt auf 90° abkühlen und fügt 4 g der Verbindung (9) zu. Anschliessend gibt man während 30 Min. 2,5 ml Benzonitril zu. Man lässt eine Stunde rühren und neutralisiert durch Zutropfen eines Essigsäure/Methanol-Gemisches. Die Reaktionssuspension wird auf 60° abgekühlt und filtriert. Man wäscht mit Methanol, Wasser und nochmals Methanol und trocknet im Vakuum.

Das erhaltene Pigment der Formel (12)

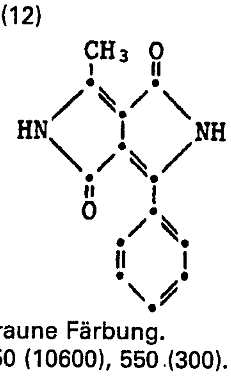

(12)

ergibt, in PVC eingarbeitet, eine gelb-braune Färbung.

VIS (NMP[1], $\lambda_{max}$, ε): 433 (12000), 450 (10600), 550 (300).

| | | | | |
|---|---|---|---|---|
| $C_{13}H_{10}H_2O_2$ | Ber.: | C 69,01 | H 4,46 | N 12,38 |
| | Gef.: | C 68,50 | H 4,47 | N 12,25. |

[1]NMP = N-Methyl-pyrrolidon.

Beispiel 4

400 mg Natrium und 20 mg des Na-Salzes des Sulfobernsteinsäure-bis-2-äthylhexylesters werden bei Rückflusstemperatur in 15 ml tert.-Amylalkohol bis zur vollständigen Umsetzung geführt.

Zur klaren Lösung fügt man 1,58 g 4-Thiophenyl-benzonitril und gibt während 20 Min. in kleinen Portionen 1,68 g des Pyrrolinons der Formel (13)

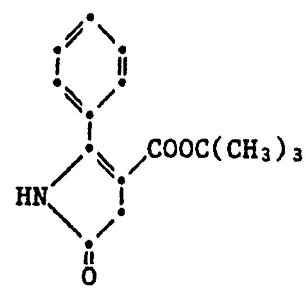

(13)

zu. Die Temperatur wird zwischen 90 und 100° gehalten. Das dunkelrote Reaktionsgemisch wird 30 Min. bei 95° gerührt und darauf durch Zutropfen von 8 ml Eisessig in 20 ml tert.-Amylalkohol sauer gestelt. Man kocht weitere 1,5 Stunden bei 100° und filtriert anschliessend bei 70°. Man wäscht farblos mit Methanol und Wasser, trocknet bei 60° im Vakuum und erhält so 1,47 g (74% d.Th.) reines Pigment der Formel (14)

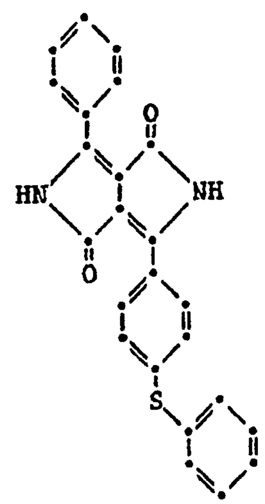

(14),

das PVC in roten Tönen färbt.

UV/VIS (in DMF[2], $\lambda_{max}$, $\varepsilon$): 350 (11500), 480 (28400), 518 (36800).

| | | | | | |
|---|---|---|---|---|---|
| $C_{24}H_{16}N_2O_2S$ | Ber.: | C 72,71 | H 4,07 | N 7,07 | S, 8,09 |
| | Gef.: | C 72,06 | H 4,12 | N 7,11 | S, 8,01. |

Die Herstellung des Pyrrolidons der Formel (13) wird im Beispiel 9 beschrieben.

Beispiel 5

Man geht gleich vor wie in Beispiel 4. Statt 4-Thiophenylbenzonitril gibt man Terephthalonitril zu und führt die Reaktion bei 85° durch. Das Pigment der Formel (15)

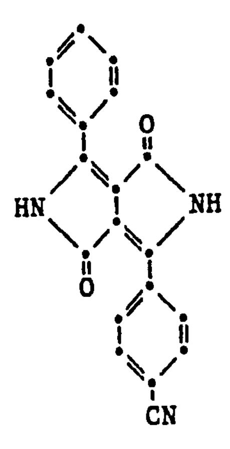

(15)

[2]DMF = Dimethylformamid

wird in 80%iger Ausbeute isoliert; es ergibt, in PVC eingearbeitet, eine rote Färbung.

Absorptionsspektrum in DMF: $\lambda_{max}$ [nm]: 271, 310, 485, 520.

| | | | | |
|---|---|---|---|---|
| $C_{19}H_{11}N_3O_2$ | Ber.: | C 72,84 | H 3,54 | N 13,41 |
| | Gef.: | C 72,15 | H 3,64 | N 13,40. |

Beispiel 6

1,55 g Natrium und 0,02 g Sulfobernsteinsäure-bis-2-äthylhexylester Natriumsalz (Emulgator) werden bei Rückflusstemperatur in 27 ml tert.-Amylalkohol bis zur vollständigen Umsetzung gerührt. Zur klaren Lösung gibt man bei 100° 6 g 4-Chlorbenzonitril und anschliessend während 30 Min. portionweise 5,1 g der Verbindung der Formel (16)

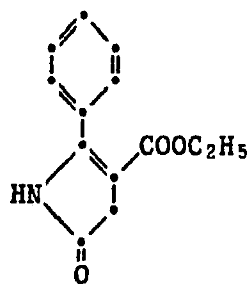

(16),

deren Herstellung im Beispiel 7 beschrieben ist.

Das Reaktionsgemisch wird eine Stunde bei 100° geführt und auf 200 ml kaltes Wasser ausgegossen. Man lässt eine Stunde bei Rückflusstemperatur rühren und leitet anschliessend zur Entfernung des organischen Lösungsmittels während einer Stunde Wasserdampf ein. Die Pigmentsuspension wird filtriert und der Filterkuchen im Vakuum bei 80° getrocknet. Man erhält 7,2 g (74% d.Th.) Pigment der Formel (17)

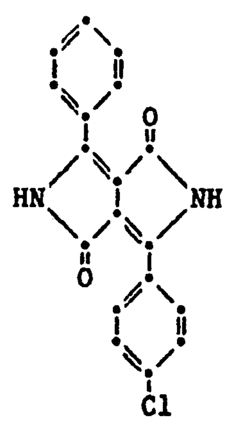

(17),

das, in PVC eingearbeitet, eine rote Färbung ergibt.

VIS (NMP, $\lambda_{max}$, ε): 471 (25300), 510 (34100)

| | | | | |
|---|---|---|---|---|
| $C_{18}H_{11}N_2O_2Cl$: | Ber.: | C 66,98 | H 3,44 | N 8,68 |
| | Gef.: | C 66,92 | H 3,60 | N 8,54. |

Beispiel 7

100 g Benzoylbernsteinsäurediäthylester und 111 g Ammoniumacetat werden in 300 ml Eisessig wärhrend 16 Stunden am Rückfluss gekocht. Das Reaktionsgemisch wird auf 3 l kaltes Wasser ausgegossen. Es bildet sich ein kristalliner Niederschlag, der abgenutscht und mit 500 ml Wasser gewaschen wird. Das Rohprodukt wird in Methylenchlorid umkristallisiert und man isoliert 48,9 g der Verbindung der Formel (16) in Form von Kristallen.

Smp.: 174°.

| | | | | |
|---|---|---|---|---|
| $C_{13}H_{13}NO_3$ | Ber.: | C 67,52 | H 5,67 | N 6,06 |
| | Gef.: | C 67,00 | H 5,71 | N 5,97. |

Beispiel 8

In einem durch eine Mischung von Trockeneis und Isopropanol auf −78° gekühlten Reaktionsgefäss werden unter Stickstoffatmosphäre 70 ml wasserfreies Tetrahydrofuran mit 13,8 ml einer 1,6-molaren Lösung n-Butyllithium in Hexan und 3,1 ml Diisopropylamin versetzt. Nach 20 Min. gibt man 2,3 g Bernsteinsäure-di-t-butylester in 5 ml Tetrahydrofuran zu und rührt wärhend 50 Min. Dann werden 10 ml

einer molaren Zinkchloridlösung in Tetrahydrofuran zugefügt und nach 30 Min. wird das Reaktionsgemisch mit 2,1 g Benzonitril versetzt. Nach 2 Std. lässt man auf Raumtemperatur erwärmen und giesst das Reaktionsgemisch auf 200 ml Wasser. Das Gemisch wird in Essigester aufgenommen, die organische Phase mit konz. Kochsalzlösung gewaschen, mit Natriumsulfat getrocknet und das Lösungsmittel am Rotationsverdampfer entfernt. Durch Chromatographie an Kieselgel mit Hexan/Essigester 8:1 erhält man 1,96 g (59% d.Th., bezogen auf den Bernsteinsäure-di-t-butylester) der Verbindung der Formel (18).

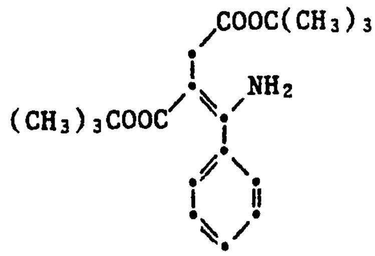

(18)

oder des entsprechenden Z-Isomeren als farblose Kristalle.

Smp.: 112—114°; UV ($CH_3OH$, $\lambda_{max}$, ε): 222 (6750), 288 (13759).

| | | | | |
|---|---|---|---|---|
| $C_{19}H_{27}NO_4$ | Ber.: | C 68,44 | H 8,16 | N 4,20 |
| | Gef.: | C 68,37 | H 8,16 | N 4,21. |

Beispiel 9

5,03 g der gemäss Beispiel 8 erhaltenen Verbindung der Formel (18) (oder des entsprechenden Z-Isomeren) werden mit 60 ml Methanol und 30 ml 30% Natriummethylat in Methanol versetzt und unter Stickstoffatmosphäre während 40 Min. auf 60° erwärmt.

Man giesst das Reaktionsgemisch auf Essigester, neutralisiert mit 1N Salzsäure und wäscht mit konz. Kochsalzlösung. Die organische Phase wird mit Natriumsulfat getrocknet und am Rotationsverdempfer eingeengt. Durch Chromatographieren an Kieselgel mit Toluol/Essigester 4:1 erhält man 2,34 g (60% der Theorie) der kristallinen Verbindung der Formel (13)

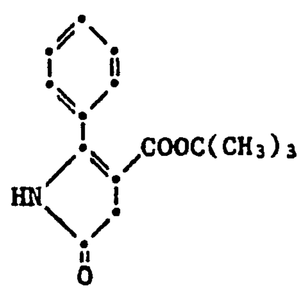

(13)

vom Smp. 153—154°.

| | | | | |
|---|---|---|---|---|
| $C_{15}H_{17}NO_3$ | Ber.: | C 69,48 | H 6,61 | N 5,40 |
| | Gef.: | C 69,22 | H 6,61 | N 5,32. |

Beispiel 10

Eine Suspension von 3,3 g 3-Aethoxycarbonyl-2-phenyl-2-pyrrolin-5-on der Formel (16), 2,5 g 2-Chlorbenzonitril und 3,6 g Kalium-tert.-butylat in 30 ml tert.-Butanol wird 5 Stunden bei Rückfluss erhitzt. Das Reaktionsgemisch wird auf Raumtemperatur abgekühlt, mit 50 ml Methanol und danach mit 3,0 ml Eisessig versetzt. Man lässt die Pigmentsuspension 5 Min. rühren, nutscht sie ab und rückflussiert zur Reinigung das rohe Pigment in 50 ml Methanol während 16 Stunden. Das Pigment wird abgenutscht, mit Methanol gewaschen und im Vakuum bei 70° getrocknet. Man erhält 2,2 g des Pigmentes der Formel

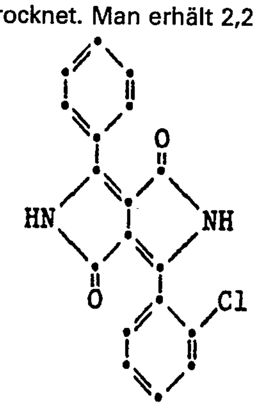

das, in PVC eingearbeitet, eine rotorange Färbung ergibt VIS (NMP, $\lambda_{max}$, ε): 459 (14700).

| C, H, N-Analyse: | C | H | N |
|---|---|---|---|
| gef. | 66,66 | 3,60 | 8,65. |

Beispiel 11

1,4 g Natrium und 0,03 g Sulfobernsteinsäure-bis-2-äthylhexylester Natriumsalz (Emulgator) werden bei Rückflusstemperatur in 20 ml tert.-Amylalkohol bis zur vollständigen Umsetzung gerührt. Zur klaren Lösung gibt man 1,76 g Laurinsäurenitril und anschliessend in zwei Portionen innerhalb von 5 Min 2,0 g der Verbindung der Formel

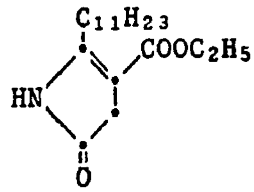

Das Reaktionsgemisch wird während 2 Std. am Rückfluss gekocht und darauf auf 100 ml Eis/Wasser ausgegossen. Man neutralisiert mit 1N HCl, filtriert das ausgefällte Pigment ab, und wäscht mit Wasser und Methanol. Das erhaltene gelbe Produkt der Formel

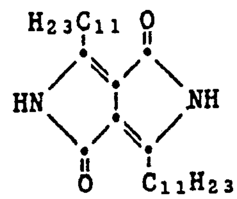

wird im Vakuum bei 50° getrocknet.

Smp. 247—250°.

UV/VIS (NMP $\lambda_{max}$, ε); 385 (10400), 402 (12000).

| | | | |
|---|---|---|---|
| $C_{28}H_{48}N_2O_2$ Ber.: | C 75,63 | H 10,88 | N 6,30 |
| Gef.: | C 75,39 | H 10,73 | N 6,28. |

Beispiel 12

Man geht vor wie in Beispiel 1, anstelle von Acetonitril wird jedoch Isobutyronitril (1,5 Aequivalente) eingesetzt. Man isoliert ein gelbes Pigment der Formel

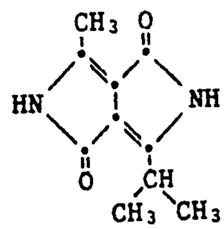

in einer Ausbeute von 11%.

UV/VIS ($CH_3OH$, $\lambda_{max}$, ε): 383 (13400), 397 (14800).

| | | | |
|---|---|---|---|
| $C_{20}H_{12}N_2O_2$ Ber.: | C 62,49 | H 6,29 | N 14,57 |
| Gef.: | C 61,76 | H 6,37 | N 14,00. |

Beispiel 13

Man verfährt wie in Beispiel 1, setzt aber statt Acetonitril Laurinsäurenitril (2 Aequivalente) ein. Das gelbe Pigment der Formel

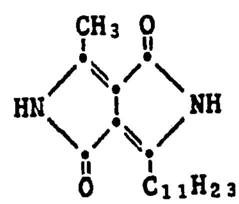

wird in einer Ausbeute von 10% isoliert.

UV/VIS ($CH_3OH$, $\lambda_{max}$, ε): 382 (14750), 398 (16250).

| | | | |
|---|---|---|---|
| $C_{18}H_{28}N_2O_2$ Ber.: | C 71,02 | H 9,27 | N 9,20 |
| Gef.: | C 71,11 | H 9,41 | N 9,04. |

## Beispiel 14

Man verfährt gleich wie in Beispiel 6, setzt aber statt 4-Chlorbenzonitril p-Tolunitril ein. Es wird mit 49% Ausbeute ein Pigment der Formel

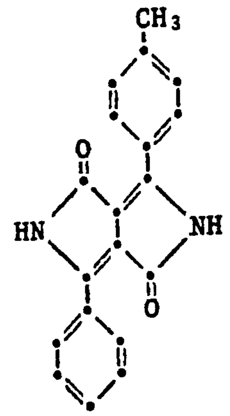

isoliert, das in PVC eine rote Färbung ergibt.

UV/VIS (NMP, $\lambda_{max}$, ε): 307 (14000), 312 (14000), 472 (26300), 507 (35600).

| | | | |
|---|---|---|---|
| $C_{19}H_{14}N_2O_2$ Ber.: | C 75,48 | H 4,67 | N 9,27 |
| Gef.: | C 75,30 | H 4,70 | N 9,23. |

## Beispiel 15

500 mg Natrium und 20 mg Sulfobernsteinsäure-bis-2-äthylhexylester Natriumsalz (Emulgator) werden bei Rückflüsstemperatur in 20 ml tert.-Amylalkohol bis zur vollständigen Umsetzung gerührt. Man gibt 1,75 ml Butyronitril und anschliessend über 3 Min in kleinen Portionen 1,46 g der Verbindung der Formel

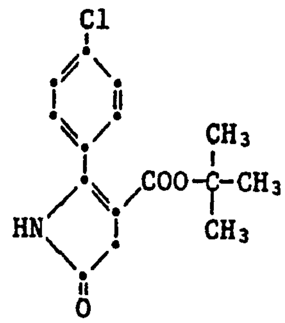

zu. Das Gemisch wird während 1 Stunde am Rückfluss gekocht und darauf auf 70° abgekühlt. Man lässt eine Mischung von 10 ml Eisessig und 15 ml Methanol zutropfen und rührt eine weitere Stunde bei 80°. Das ausgefällte Pigment wird abfiltriert und mit Methanol gewaschen. Nach Trocknen im Vakuum bei 80° erhält man 750 mg (52% d.Th.) eines Pigmentes der Formel

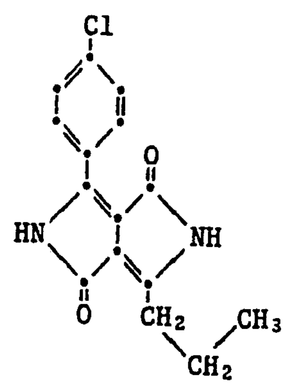

das in PVC eingearbeitet eine orange Färbung ergibt.

VIS (DMF, $\lambda_{max}$, ε): 440 (11100), 460 (10200), 510 (sh), 630 (600).

| | | | | | |
|---|---|---|---|---|---|
| $C_{15}H_{13}N_2O_2Cl$ | Ber.: | C 62,40 | H 4,54 | N 9,70 | Cl, 12,28 |
| | Gef.: | C 62,28 | H 4,54 | N 9,70 | Cl 12,30. |

Beispiel 16

Man verfährt gleich wie in Beispiel 15, setzt aber statt Butyronitril 4-Cyan-Biphenyl ein. Nach dem Trocknen im Vakuum (60°) wird das Pigment der Formel

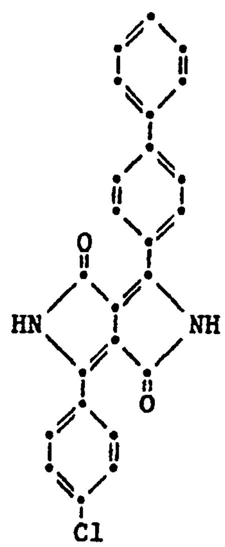

das PVC rot färbt, in einer Ausbeute von 70% d.Th. isoliert.

UV/VIS (NMP, $\lambda_{max}$, ε): 336 (16800), 488 (31000), 524 (40000).

| | | | | | |
|---|---|---|---|---|---|
| $C_{24}H_{15}N_2O_2Cl$ | Ber.: | C 72,27 | H 3,79 | N 7,02 | Cl, 8,89 |
| | Gef.: | C 70,54 | H 3,83 | N 6,81 | Cl, 8,70. |

Beispiel 17

Bei gleichem Verfahren wie in Beispiel 15 wird statt Butyronitril Isophthalonitril eingesetzt. Man rekristallisiert das Rohprodukt in NMP und erhält mit 87% d.Th. ein Pigment der Formel

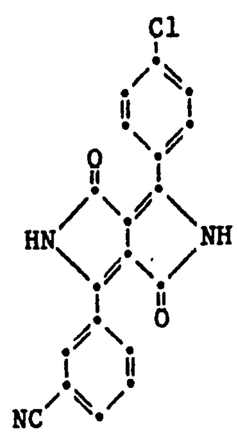

das in PVC eine rote Färbung ergibt.

UV/VIS (NMP, $\lambda_{max}$, ε): 288 (14800), 308 (14200), 450 (sh), 480 (23600), 513 (31000).

| | | | | | |
|---|---|---|---|---|---|
| $C_{10}H_{10}N_3O_2Cl$ | Ber.: | C 65,62 | H 2,90 | N 12,08 | Cl 10,19 |
| | Gef.: | C 65,11 | H 2,96 | N 11,96 | Cl 10,12. |

Beispiel 18

1,7 g Natrium und 0,1 g Sulfobernsteinsäure-bis-2-äthylhexylester Natriumsalz (Emulgator) werden be

Rückflusstemperatur in 30 ml tert.-Amylalkohol bis zur vollständigen Umsetzung gerührt. Man gibt bei 90° 5,1 ml Benzonitril und anschliessend über 10 Min in kleinen Portionen 8,95 g der Verbindung der Formel

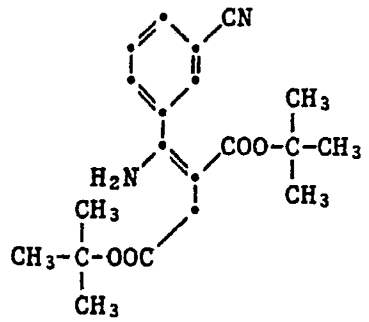

zu. Das Gemisch wird während 1 Stunde bei 90° gekocht und darauf auf 100 ml Wasser ausgegossen. Nach einer einstündigen Wasserdampfdestillation filtriert man das Reaktionsgemisch und reinigt das erhaltene Rohprodukt durch Rekristallisation in DMF während 5 Stunden bei 120°. Die Suspension wird abgekühlt, filtriert und mit Methanol und Wasser gewaschen. Man erhält 4,8 g (61% d.Th.) eines Pigmentes der Formel

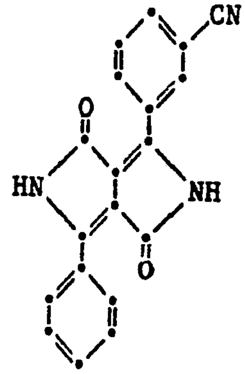

das in PVC eingearbeitet eine rote Färbung ergibt.

UV/VIS (NMP, $\lambda_{max}$, $\varepsilon$): 289 (14600), 305 (sh), 455 (sh), 478 (21800), 512 (29600).

| | | | | |
|---|---|---|---|---|
| $C_{19}H_{11}N_3O_2$ | Ber.: | C 72,84 | H 3,54 | N 13,41 |
| | Gef.: | C 72,19 | H 3,65 | N 13,13. |

Beispiel 19

Man geht gleich vor wie in Beispiel 18, verwendet jedoch statt Benzonitril Terephthalonitril. Nach einer Rekristallisation in NMP wird in 27% Ausbeute ein Pigment der Formel

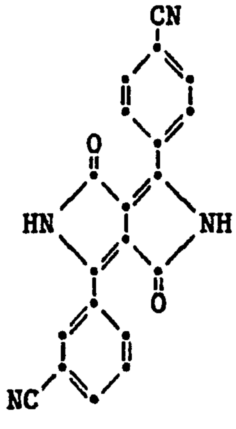

isoliert, das, in PVC eingearbeitet, eine rote Färbung ergibt.

UV/VIS (NMP, $\lambda_{max}$, $\varepsilon$): 280 (31600), 310 (sh), 490 (20600), 521 (24600).

| | | | | |
|---|---|---|---|---|
| $C_{20}H_{10}N_4O_2$ | Ber.: | C 71,00 | H 2,98 | N 16,56 |
| | Gef.: | C 69,08 | H 3,11 | N 16,03. |

Beispiel 20

2,2 g der Verbindung der Formel

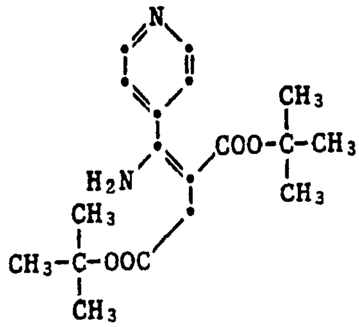

1,8 g p-Chlorbenzonitril und 2,22 g Kalium-tert.-butoxid werden in 30 ml tert.-Butanol während 2½ Stunden am Rückfluss gekocht. Das Reaktionsgemisch wird auf 150 ml Wasser ausgegossen, mit 65 ml Methanol und 25 ml 1N Salzsäure versetzt und während 1 Stunde bei Zimmertemperatur gerührt. Der kristalline Niederschlag wird mit Wasser und Methanol gewaschen und im Vakuum bei 60° getrocknet. Man erhält 1,6 g (76% d.Th. bezogen auf Enaminodiester) rotes Pigment der Formel

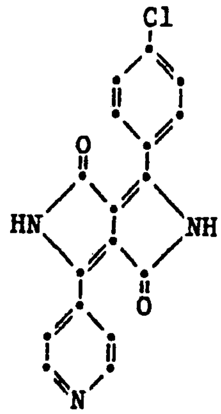

UV/VIS (NMP, $\lambda_{max}$, $\varepsilon$): 268 (26660), 308 (12340), 450 (sh), 483 (21360), 517 (26900).

| | | | | |
|---|---|---|---|---|
| $C_{17}H_{10}N_3O_2Cl$ | Ber.: | C 63,07 | H 3,11 | N 12,98 |
| | Gef.: | C 62,64 | H 3,33 | N 12,56. |

Beispiel 21

Zu einer Lösung von 6,6 g 3-Aethoxycarbonyl-2-phenyl-2-pyrrolin-5-on der Formel (16) und 5,6 g 1-Naphthonitril in 60 ml tert.-Butanol werden 10,8 g Kalium-tert.-butoxid zugegeben und das Reaktionsgemisch 5 Stunden bei Rückfluss gerührt. Uebliche Aufarbeitung liefert 3,0 g (28%) Pigment der Formel

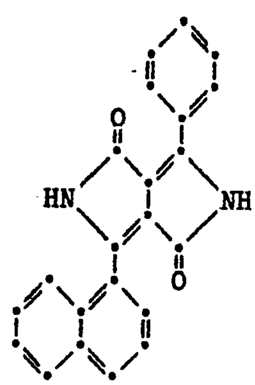

das in PVC eingearbeitet, eine orange Färbung ergibt.

VIS (NMP, $\lambda_{max}$, $\varepsilon$): 470 (15800), 493 (16300).

| C, H, N-Analyse: | C | H | N |
|---|---|---|---|
| gef. | 77,52 | 4,28 | 8,28%. |

Beispiel 23

Gemäss Beispiel 21 werden 4,3 g Laktam der Formel (16), 2,8 o-Tolunitril und 4,8 Kalium-tert.-butoxid in 40 ml tert.-Butanol 5 Stunden bei Rückfluss gerührt. Uebliche Aufarbeitung liefert 0,7 g Pigment der Formel

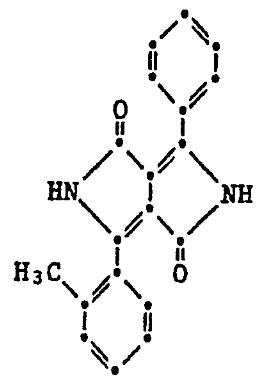

das in PVC eingearbeit, eine gelborange Färbung ergibt.

VIS (NMP, $\lambda_{max}$, ε): 453 (19000), 481 (21100).

| C, H, N-Analyse: | C | H | N |
|---|---|---|---|
| gef. | 73,77 | 4,84 | 8,73%. |

Beispiel 24

Gemäss Beispiel 21 werden 4,3 g Laktam der Formel (16), 3,4 g 2,5-Dimethylbenzonitril und 4,8 g Kalium-tert.-Butoxid in 30 ml tert.-Butanol bei Rückfluss gerührt. Uebliche Aufarbeitung liefert 0,4 g (6%) Pigment der Formel

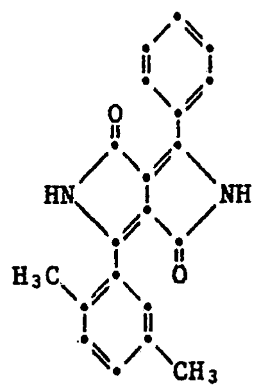

das, in PVC eingearbeitet, eine gelbe Färbung ergibt.

VIS (NMP, $\lambda_{max}$, ε): 456 (18400), 482 (20600).

| C, H, N-Analyse: | C | H | N |
|---|---|---|---|
| gef. | 74,92 | 5,17 | 8,59%. |

Beispiel 25

Gemäss Beispiel 21 werden 3,3 g Laktam der Formel (16), 2,1 g Benzylcyanid und 3,6 g Kalium-tert.-butoxid in 30 ml tert.-Butanol 2 Stunden bei Rückfluss gerührt. Uebliche Aufarbeitung liefert 0,8 g (17%) Pigment der Formel

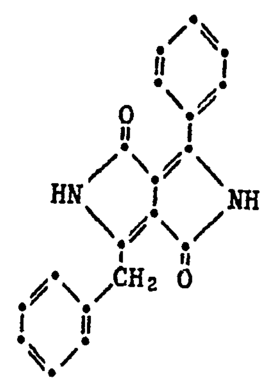

das, in PVC eingearbeitet, eine orange Färbung ergibt.

VIS (NMP, $\lambda_{max}$, ε): 381 (11600), 466 (1400).

| C, H, N-Analyse: | C | H | N |
|---|---|---|---|
| gef. | 75,19 | 4,64 | 9,23%. |

Beispiel 26

Gemäss Beispiel 21 werden 4,3 g Laktam der Formel (16), 3,1 g 3-Phenyl-propionitril und 4,8 g Kalium-tert.-butoxid in 30 ml tert.-Butanol 5 Stunden bei Rückfluss gerührt. Uebliche Aufarbeitung liefert 1,0 g Pigment der Formel

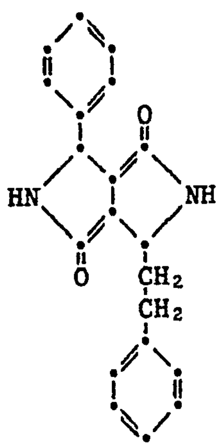

das, in PVC eingearbeitet eine orange Färbung ergibt.

VIS (NMP, $\lambda_{max}$, ε): 438 (15800), 459 (14600).

| C, H, N-Analyse: | C | H | N |
|---|---|---|---|
| gef. | 75,24 | 5,14 | 8,72%. |

Beispiel 27

(Anwendung in Weich-Polyvinylchlorid)

0,6 g des gemäss Beispiel 2 erhaltenen Pigments werden mit 67 g Polyvinylchlorid, 33 g Dioctylphthalat, 2 g Dibutylzinndilaurat und 2 g Titandioxyd zusammengemischt und auf dem Walzenstuhl während 15 minuten bei 160°C zu einer dünnen Folie verarbeitet. Die so erzeugte gelbe Färbung ist farbstark und zeichnet sich durch gute Pigmenteigenschaften aus.

Beispiel 28

(Anwendung in Polyäthylen)

0,2 g des gemäss Beispiel 10 erhaltenen Pigmentes, 1 g Titandioxyd (Rutil) und 100 g LD-Polyäthylengranulat werden in einer Trommel gemischt und das Gemisch anschiessend auf dem Mischwalzwerk bei 130° verarbeitet. Die Masse wird heiss zu Platten verpresst oder in der Strangpresse verformt. Die Platten zeigen eine schöne rote Färbung von guter Lichtechtheit.

Beispiel 29

(Anwendung in Alkyd-Melamin-Einbrennlack)

60 g einer 60%igen Lösung eines nicht-trocknenden Alkydharzes in Xylol (Hendelsname Beckosol 27-320® der Firma Reichhold-Albert-Chemie), 36 g einer 50%igen Lösung eines Melamin-Formaldehyd-Harzes in einem Butanol-Xylol-Gemisch (Handelsname Super-Beckamin 13-501® der Firma Reichhold-Albert-Chemie), 2 g Xylol und 2 g Methylcellosolve werden vermischt und 100 g dieses Gemisches werden mit Hilfe eines Rührers zu einer homogenen Lacklösung verrührt.

95 g des so erhaltenen Klarlackes und 5 g des gemäss Beispiel 5 erhaltenen Pigmentes werden in einer Kugelmühle während 72 Stunden gemahlen. Der eingefärbte Lack wird dann nach üblicher Spriztmethode auf Blech appliziert und 30 Minuten bei 120°C eingebrannt. Man erhält eine rote Lackierung von guter Lichtechtheit.

**Patentansprüche**

1. Verfahren zur Herstellung von Pyrrolo-[3,4-c]-pyrrolen der Formel (1)

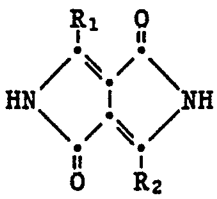

(1),

worin $R_1$ und $R_2$ $C_1$—$C_{18}$-Alkyl, Cyclohexyl, Benzyl, Phenyläthyl, Diphenylyl, Naphthyl, einen heterocyclischen Rest aus der Gruppe bestehend aus Pyridyl, Pyrimidyl, Pyrazinyl, Triazinyl, Furanyl, Pyrrolyl, Thiophenyl, Chinolyl, Cumarinyl, Benzfuranyl, Benzimidazolyl und Benzoxazolyl oder eine Gruppe

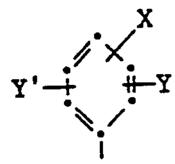

bedeuten, worin X, Y und Y' H- oder Halogenatome, Carbamoyl-, Trifluormethyl-, Cyan- oder $C_2$—$C_6$-Alkylcarbamoylgruppen, Alkyl-, Alkoxy- oder Alkylmercaptogruppen mit 1—6 C, Alkoxycarbonyl- oder Alkanoylamino- oder Dialkylaminogruppen mit 2—6 C, gegebenenfalls durch Halogen, Alkyl oder Alkoxy mit 1—6 C substituierte Phenoxy-, Phenylmercapto, Phenoxycarbonyl-, Phenylcarbamoyl- oder Benzoylaminogruppen bedeuten dadurch gekennzeichnet, dass man einen Ester der Formeln (2) oder (3)

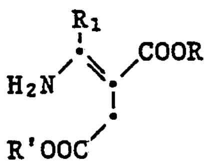

(2)

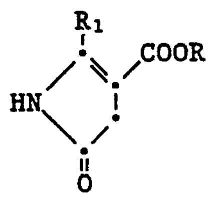

(3)

mit einem Nitril der Formel $R_2CN$ (4) in Gegenwart von 0,1 bis 10 Mol einer starken Base, bezogen auf 1 Mol des Reaktenden der Formel (2) oder (3), in einem organischen Lösungsmittel bei einer Temperatur von 60 bis 140°C umsetzt, wobei R und R' Alkylgruppen von 1—18 C-Atomen, unsubstituiertes oder durch Methoxy oder Aethoxy substituiertes Phenyl bedeuten und $R_1$ und $R_2$ die bereits angegebene Bedeutung haben.

2. Verfahren gemäss Anspruch 1, dadurch gekennzeichnet, dass man von einem Ester der Formeln (2) oder (3) ausgeht, worin R und R' $C_1$—$C_6$-Alkylgruppen bedeuten.

3. Verfahren gemäss Anspruch 1, dadurch gekennzeichnet, dass man als organisches Lösungsmittel einen sek.- oder tert.-Alkohol verwendet.

**Revendications**

1. Procédé pour préparer des pyrrolo-[3,4-c]-pyrroles de formule (1)

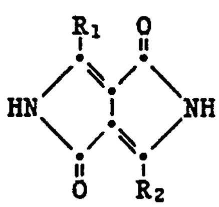

(1),

(dans laquelle $R_1$ et $R_2$ représentent, indépendamment l'un de l'autre, un groupe alkyle en $C_1$ à $C_{18}$, un groupe cyclohexyle, benzyle, phényléthyle, diphénylyle, naphtyle, un reste hétérocyclique choisi parmi

l'ensemble constitué par un groupe pyridyle, pyrimidyle, pyrazinyle, triazinyle, furannyle, pyrrolyle, thiophényle, quinoléyle, coumarinyle, benzofurannyle, benzimidazolyle et benzoxalyle, ou un groupe

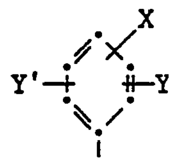

dans lequel X, Y et Y' représentent chacun un atome de H ou d'halogène, un groupe carbamoyle, trifluorométhyle, cyano ou alkyl(en $C_2$ à $C_6$) carbamoyle, un groupe alkyle, alcoxy ou alkylmercapto ayant 1 à 6 atomes de carbone, alcoxy carbonyle ou alcanoylamino ou dialkylamino ayant 2 à 6 atomes de carbone, un groupe phénoxy, phénoxymercapto, phénoxycarbonyle, phénylcarbonyle ou benzoylamino (éventuellement substitués par de l'halogène, par un groupe alkyle ou alcoxy ayant 1 à 6 atomes de carbone)), procédé caractérisé en ce qu'on fait réagir un ester répondant aux formules (2) ou (3):

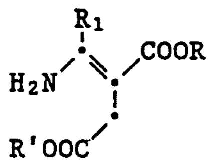

(2)

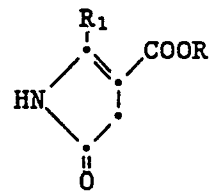

(3)

avec un nitrile de formule $R_2CN$ (4), en présence de 0,1 à 10 moles d'une base forte, pour une mole du corps de formule (2) ou (3) à faire réagir, dans un solvant organique à une température de 60 à 140°C, les symboles R et R' représentant des groupes alkyles ayant 1 à 18 atomes de carbone, un groupe phényle non substitué ou substitué par un groupe méthoxy ou éthoxy, et $R_1$ et $R_2$ ayant le sens déjà indiqué.

2. Procédé selon la revendication 1, caractérisé en ce qu'on part d'un ester répondant aux formules (2) ou (3), dans lesquelles R et R' représentent des groupes alkyles en $C_1$ à $C_6$.

3. Procédé selon la revendication 1, caractérisé en ce qu'on utilise comme solvant organique un alcool secondaire ou tertiaire.

**Claims**

1. A process for the preparation of a pyrrolo[3,4-c]pyrrole of the formula (1)

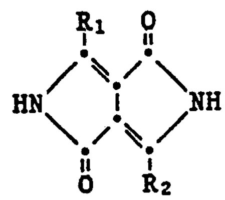

(1),

in which $R_1$ and $R_2$ are $C_1$—$C_{18}$alkkyl, cyclohexyl, benzyl, phenylethyl, biphenylyl, naphthyl, a heterocyclic radical from the group consisting of pyridyl, pyrimidyl, pyrazinyl, triazinyl, furanyl, pyrrolyl, thiophenyl, quinolyl, cumarinyl, benzofuranyl, benzimidazolyl and benzoxazolyl or a group

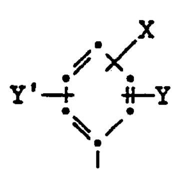

in which X, Y and Y' are H or halogen atoms, carbamoyl, trifluoromethyl, cyano or $C_2$—$C_6$alkylcarbamoyl groups, alkyl, alkoxy or alkylmercapto grous of 1—6 C, alkoxycarbonyl or alkanoylamino or dialkylamino groups of 2—6 C, phenoxy, phenylmercapto, phenoxycarbonyl, phenylcarbamoyl or benzoylamino groups

each unsubstituted or substituted by halogen, alkyl or alkoxy of 1—6 C, which process comprises reacting an ester of the formulae (2) or (3)

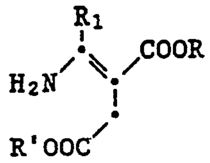

(2)

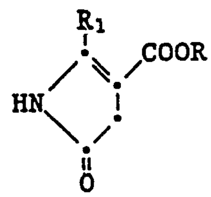

(3)

with a nitrile of the formula $R_2CN$ (4) in the presence of 0.1 to 10 mol of a strong base, based on 1 mol of the reactants of the formula (2) or (3), in an organic solvent at a temperature from 60 to 140°C, R and R′ being alkyl groups of 1—18 C atoms, phenyl which is unsubstituted or substituted by methoxy or ethoxy and $R_1$ and $R_2$ being as defined above.

2. A process according to claim 1, which comprises starting from an ester of the formulae (2) or (3) in which R and R′ are $C_1$—$C_6$alkyl groups.

3. A process according to claim 1, wherein the organic solvent used is a sec-alcohol or tert-alcohol.